# EUROPEAN PATENT SPECIFICATION

(11) **EP 2 506 011 B1**
(45) Date of publication and mention of the grant of the patent: **23.09.2015**
(21) Application number: 12161798.9
(22) Date of filing: 28.03.2012
(51) Int. Cl.: G01N 33/50, G01N 33/58, G01N 33/68

(54) **Method and kit for detecting and quantifying intracellular histamine and histamine release**
Verfahren und Kit zur Erfassung und Quantifizierung von intrazellulärem Histamin und Histaminfreisetzung
Procédé et kit de détection et de quantification d'histamine intracellulaire et déclencheur d'histamine

(30) Priority: 28.03.2011 EP 11159980
(43) Date of publication of application: 03.10.2012
(73) Proprietor: Universiteit Antwerpen, 2000 Antwerpen (BE); Universitair Ziekenhuis Antwerpen, 2650 Edegem (BE)
(72) Inventor: Bridts, Chris, 2550 Kontich (BE); Ebo, Didier, 9090 Melle (BE)
(74) Representative: Paemen, Liesbet R.J.

(56) References cited:
- JACOBI H H ET AL: "Histamine immunocytochemistry: a new method for detection of basophils in peripheral blood", JOURNAL OF IMMUNOLOGICAL METHODS, ELSEVIER SCIENCE PUBLISHERS B.V.,AMSTERDAM, NL, vol. 237, no. 1-2, 1 April 2000 (2000-04-01), pages 29-37, XP004192492, ISSN: 0022-1759, DOI: 10.1016/S0022-1759(00)00131-9
- DVORAK ANN M ET AL: "Diamine oxidase-gold ultrastructural localization of histamine in human skin biopsies containing mast cells stimulated to degranulate in vivo by exposure to recombinant human stem cell factor", BLOOD, vol. 90, no. 8, 1997, pages 2893-2900, XP055016398, ISSN: 0006-4971
- SZEBERENYI J B ET AL: "Intracellular histamine content increases during in vitro dendritic cell differentiation", INFLAMMATION RESEARCH, vol. 50, no. Supplement 2, April 2001 (2001-04), pages S112-S113, XP002667182, ISSN: 1023-3830
- DIDIER G. EBO ET AL: "Analyzing histamine release by flow cytometry (HistaFlow): A novel instrument to study the degranulation patterns of basophils", JOURNAL OF IMMUNOLOGICAL METHODS, vol. 375, no. 1-2, 1 January 2012 (2012-01-01), pages 30-38, XP055016369, ISSN: 0022-1759, DOI: 10.1016/j.jim.2011.09.003
- D. G. EBO ET AL: "Combined analysis of intracellular signalling and immunophenotype of human peripheral blood basophils by flow cytometry: a proof of concept", CLINICAL & EXPERIMENTAL ALLERGY, vol. 37, no. 11, 1 November 2007 (2007-11-01), pages 1668-1675, XP055016326, ISSN: 0954-7894, DOI: 10.1111/j.1365-2222.2007.02819.x
- MACGLASHAN D JR: "Expression of CD203c and CD63 in human basophils: relationship to differential regulation of piecemeal and anaphylactic degranulation processes", CLINICAL AND EXPERIMENTAL ALLERGY, vol. 40, no. 9, September 2010 (2010-09), pages 1365-1377, XP002667183, ISSN: 0954-7894

## Description

### FIELD OF THE INVENTION

The present invention relates to a cytometric method and a kit for quantifying intracellular histamine and histamine release in mast cells and/or basophils at single cell level.

### BACKGROUND OF THE INVENTION

Mast cells and/or basophils play a central role in the IgE-mediated allergic reaction. Upon the first encounter with an allergen, allergen-specific IgE antibodies are produced by B lymphocytes. These IgE antibodies bind to FcεRI receptors found on the surface of mast cells and/or basophils. This process is called sensitization. The next time(s) one runs across said allergen, it cross-links FcεRI-bound specific IgE antibodies on said sensitized IgE-primed mast cells and/or basophils. This cross-linking ultimately induces degranulation of said mast cells and/or basophils, which leads to the release of various potent mediators such as histamine that contribute to the allergic reaction. Alternatively, basophils can also be activated by various other stimuli such as anaphylatoxins, bacterial products, cytokines, phorbol esters, etc. The activation of basophils is further characterized by an up-regulation of the activation markers CD63 and CD203c on their membranes.
Mast cell and basophil activation was first studied by quantification of the amount of mediator release. Traditionally, histamine release has been quantified using fluorimetric and colorimetric techniques that measure its extracellular content after degranulation of activated basophils and mast cells in the supernatant. However, the time and cost consuming two-step approach i.e. (a) cell incubation and (b) quantification of low concentrations of this rapidly metabolized compound has hampered the application of such assays. Moreover, as in these techniques histamine is quantified in the supernatant, results represent only an average of (isolated) cells analyzed and do not inform on cells with heterogeneity in responses.
In 1993 Dvorak et al (J Histochem Cytochem, 41:787-800) successfully localized intracellular histamine in mast cells by applying an enzyme-affinity-gold method based on the affinity of the histaminase diamine oxidase (DAO) for its substrate. In 1994, this DAO-gold based technique was applied to localize histamine in activated basophils (Dvorak et al, 1994, Int Arch Allergy Immunol, 105:8-11; Dvorak et al, 1995, Blood, 86:3560-3566; Dvorak et al, 1996, Blood, 88:4090-4101; Dvorak et al, 1996, J Allergy Clin Immunol, 98:355-370; Dvorak et al, 1998, Prog Histochem Cytochem,33:III-320). Furthermore, Dvorak et al. (1997, Blood, 90 (8): 2893-2900) discloses the detection of histamine in skin biopsies using the DAO-conjugated gold particles.
In the early 1990ies, the discovery of the basophil activation marker CD63 induced the development of a flow cytometric technique to analyze and quantify *in vitro* activation of basophils (Knol et al, 1991, J Allergy Clin Immunol, 88:328-338). During the last decade potentials and limitations of this technique (basophil activation test: BAT) have been repeatedly reviewed (Ebo et al, 2004, Clin Exp Allergy, 34:332-339; Ebo et al, 2006, Allergy, 61:028-1039; Ebo et al, 2008, Cytometry B clin Cytom, 74:201-210). At present, the tetraspanin CD63 (gp50) of the lysosome-associated membrane protein (LAMP) family and the lineage-specific ecto-enzyme CD203c (ectonucleotide pyrophosphate/ phosphodiesterase or ENPP-3) (but also intracellular signaling molecules such as phosphorylated p38 mitogen protein kinase (MAPK) have been validated as potential read outs for flow-assisted analysis of *in vitro* activated basophils). Interestingly, it has emerged that the up-regulation of CD63 and CD203c display distinct kinetics and are regulated by different pathways. Furthermore, the relationship between both activation markers and mediator release remains still elusive. Comparative analyses between flow-assisted phenotyping and quantification of extracellular histamine has revealed that expression of these markers can (to some extent) be dissociated from mediator release (MacGlashan, 2010, Clin Exp Allergy, 40:1365-1377; MacGlashan, 1995, J Leukoc Biol, 58:177-188). However, this method requires homogeneous cell populations and results represent an average of purified cells analyzed.
Szeberenyi (2001, Inflammation Research, 50, Suppl 2: S112-S113) investigates the changes in intracellular histamine- and histidine-decarboxylase (HDC) content during in vitro dendritic cell differentiation accelerated by GM-CSF and IL-4. The flow cytometry analysis method used is disclosed in Jacobi et al (2000).
Jacobi et al. (2000 J; Immunol Method 237:29-37) describe the detection of intracellular histamine using immunohistochemistry (detected either microscopically or through flow cytometry). However, the authors indicate that the fixation and subsequent permeabilization required for intracellular detection results in a significant amount of debris. They further indicate that this technique does not allow a reliable quantification of histamine and does not reliably distinguish between activated and non-activated basophils.
Accordingly, there remains a need for improved methods for measuring the activation of basophils and/or mast cells.

### SUMMARY OF THE INVENTION

The present invention provides methods for quantifying intracellular histamine and histamine release in individual mast cells and/or basophils. More particularly, the inventors have found that intracellular histamine and histamine release can successfully be analyzed and quantified using cytometric methods applying an enzyme-affinity-fluorochrome method based on the affinity of the histaminase diamine oxidase (DAO) for its substrate. An important advantage of this technology is that quantification of histamine can occur intracellularly, without needing to activate the cells to ensure histamine release. Moreover, the combination with cytometric methods allows a selective quantification of histamine from basophils and/or mast cells. Moreover the methods of the invention allow a differentiation in the type of activation (i.e. piecemeal or anaphylactic) of the cells, which can be of interest both in a scientific and in a therapeutic context.

In a first aspect the invention thus provides methods for quantifying intracellular histamine and histamine release in individual mast cells and/or basophils. In particular embodiments, the methods of the invention comprise the steps of:
a) fixing said mast cells and/or basophils with a fixation buffer comprising a fixation reagent;
b) permeabilizing said mast cells and/or basophils with a permeabilization buffer comprising a permeabilization agent;
d) staining said mast cells and/or basophils intracellularly with fluorochrome-labeled histaminase diamine oxidase; and
e) analyzing said stained mast cells and/or basophils on a cytometer,
wherein the signal from said fluorochrome-labeled histaminase diamine oxidase is indicative of the quantity of histamine present per cell in each of said mast cells and/or basophils.
The methods of the invention are suitable for use on isolated mast cells and basophils, subpopulations comprising mast cells and/or basophils cell lines or biological samples. Biological samples which can be analysed include but are not limited to a blood sample, a tissue biopsy, e.g. a skin biopsy. In particular embodiments of the present invention, the mast cells and/or basophils are provided as an isolated (pure) population of mast cells and/or basophils. In further particular embodiments, the methods of the invention are characterized in that they do not require the prior activation of the mast cells and/or basophils in order to allow histamine quantification.
Where the sample is not an isolated (pure) mast cell and/or basophil population but contains other cells than mast cells and basophils (e.g. a biological sample), identification and optionally selection of the mast cells and/or basophils in the sample is desirable.
In particular embodiments of the methods of the present invention, the mast cells and/or basophils are identified by using selection marker. Thus, in these embodiments, the methods of the invention for quantifying intracellular histamine and histamine release in individual mast cells and/or basophils in a sample comprise the steps of:
a) staining said mast cells and/or basophils for one or more selection markers; and further
b) fixing said mast cells and/or basophils with a fixation buffer comprising a fixation reagent;
c) permeabilizing said mast cells and/or basophils with a permeabilization buffer comprising a permeabilization agent;
d) staining said mast cells and/or basophils intracellularly with fluorochrome-labeled histaminase diamine oxidase; and
e) analyzing said stained mast cells and/or basophils on a cytometer,
wherein the signal from said fluorochrome-labeled histaminase diamine oxidase is indicative of the quantity of histamine present per cell in each of said mast cells and/or basophils.
In particular embodiments, the cellular membrane selection marker is identified using an antibody directed against the marker. More particularly, the antibody is labeled with a fluorochrome, whereby the fluorochrome has a distinct emission spectrum separated spectrally from the fluorochrome-labeled histaminase diamine oxidase. Different cellular membrane selection markers for basophils and/or mast cells are envisaged for use in the methods of the present invention. In particular embodiments, the one or more selection markers can comprises IgE, or the IgE receptor, CD203c, etc..
The methods of the invention further allow to further characterize activated cell populations of mast cells as to their histamine content and/or release. For this purpose, the cells can be stained with an activation marker. Thus, in particular embodiments of the methods of the present invention, the one or more activation markers comprise activation markers such as CD63 or CD203c, etc..
Additionally or alternatively, where the sample contains cells other than basophils and/or mast cells, the basophil and/or mast cell population is further purified by removing the cell types other than basophils and/or mast cells. For instance, where the sample may contain red blood cells, a lysing reagent can be used, to selectively lyse red blood cells. The lysing reagent can be present in the fixation buffer or the method of the invention can comprise a separate step wherein the cells are treated with lysing buffer comprising a lysing reagent.
As indicated above, the methods of the present invention make use of a fixation buffer and a permeabilization buffer. In particular embodiments of the methods of the present invention said fixation buffer comprises formaldehyde as a fixating reagent. In particular embodiments of the methods of the present invention said permeabilization buffer comprises Triton-X-100. In particular embodiments, the fluorochrome used to label histamine diamine oxidase is phycoerythrin.
In particular embodiments, the methods of the present invention further comprise the step of comparing the signal from said fluorochrome-labeled histaminase diamine oxidase to a reference value (e.g, obtained from resting cells), wherein said reference value can be used to evaluate degranulation of said mast cells and basophils.
In further particular embodiments, the methods of the invention include a calibration of the amount of intracellular histamine detected. This is ensured by adding calibration particles labeled with said fluorochrome of said fluorochrome-labeled histaminase diamine oxidase to said mast cells and basophils.
The methods of the present invention are particularly suitable for quantification of intracellular histamine and histamine release in mast cells and/or basophils, more particularly in individual mast cells and/or basophils.
In particular embodiments, the methods of the invention can be used to determine the effect of a test compound on histamine content and/or release of mast cells and basophils. In these embodiments, the methods of the invention comprise the step of incubating said mast cells and basophils at optimal temperature conditions with a test compound prior to mixing said cells with fixation buffer, and further carrying out the method steps as described herein. Comparison of a sample with and without the test compound will allow determination of whether or not the test compound influences the histamine content and/or release by said mast cells and/or basophils.
In particular embodiments the methods of the invention further comprise the step of incubating mast cells and basophils with an activator. Examples of suitable activators include but are not limited to anti-IgE antibody, N-formyl-met-leu-phe (fMLP) or phorbol 12-myristate 13-acetate/ionomycin (PMA/ionomycin). The activator is incubated with the cells, prior to mixing the cells with fixation buffer.

In another aspect, the present invention provides kits for quantifying intracellular histamine and histamine release in individual mast cells and basophils by flow cytometry comprising:
- a fluorochrome-labeled histaminase diamine oxidase;
- one or more fluorochrome-labeled antibodies directed against a selection marker, more particularly a cellular membrane marker for basophils and mast cells;
- fixation buffer comprising a fixation reagent; and
- permeabilization buffer comprising a permeabilization reagent.
In particular embodiments of the kits of the invention the fixation buffer comprises formaldehyde as a fixation reagent. In particular embodiments of the kits of the invention said fixation buffer comprises a lysing reagent, more particularly a lysing reagent for red blood cells. In particular embodiments of the kits of the present invention said permeabilization buffer comprises Triton-X-100.
Typically, the fluorochrome label present on said histaminase diamine oxidase present in the kits of the present invention is different from the fluorochrome present on the antibody directed against the selection marker. In particular embodiments of the kits of the present invention said histaminase diamine oxidase is labeled with phycoerythrin.
In particular embodiments the kits of the present invention further comprise instructions for performing the methods as described herein.
In particular embodiments the kits of the present invention further comprise calibration particles labeled with the fluorochrome of said fluorochrome-labeled histaminase diamine oxidase. The calibration particles are optionally fluorochrome-labeled calibration particles comprising the fluorochrome at quantitated levels;
In particular embodiments the kits of the present invention comprise one or more fluorochrome-labeled antibodies directed against an activation marker, preferably CD63 or CD203c.
In particular embodiments the kits of the present invention further comprise an activator for basophils and/or mast cells. Suitable activators include but are not limited to anti-IgE antibody, N-formyl-met-leu-phe (fMLP) or phorbol 12-myristate 13-acetate/ionomycin (PMA/ionomycin).
Yet another aspect of the present invention relates to the uses of the kits and the methods of the present invention for different applications.
In particular embodiments the kit is used for quantifying intracellular histamine and histamine release in individual mast cells and basophils by flow cytometry. More particularly it is envisaged that the kit is used for quantifying degranulation of individual mast cells and basophils by cytometry. In further embodiments the kit is used for quantifying the histamine content of individual mast cells and basophils by cytometry. In further embodiments the methods and kits of the invention are used to characterize activated basophils and mast cells.
In particular embodiment, the invention relates to the use of the kits and methods of the invention for in vitro determination of allergenic reaction. More particularly the methods and kits can be used as an *in vitro* allergy test.
In particular embodiments, the allergy is an allergy to one or more compounds selected from curarising neuromuscular blocking agents, antibiotics and narcotics.
In particular embodiments the invention relates to the use of the kits and methods of the invention for determining the effect of allergen immunotherapy.
In particular embodiments the invention relates to the use of the kits and methods of the invention for testing whether a compound has an anti-allergenic effect.

In particular embodiments, the invention relates to the use of the kits and methods of the invention for determining whether a compound has an effect on histamine content and/or release by basophils and/or mast cells. In further particular embodiments, the tests can be used to determine whether a compound has a histamine releasing effect. The present invention further provides the use of the method disclosed herein for high-throughput screening of the histamine releasing effect of compounds or for screening the ability of compounds to counter an allergenic reaction.

### BRI EF DESCRIPTION OF THE FIGURES

The present invention is illustrated by the following figures which are to be considered for illustrative purposes only and in no way limit the invention to the embodiments disclosed therein:

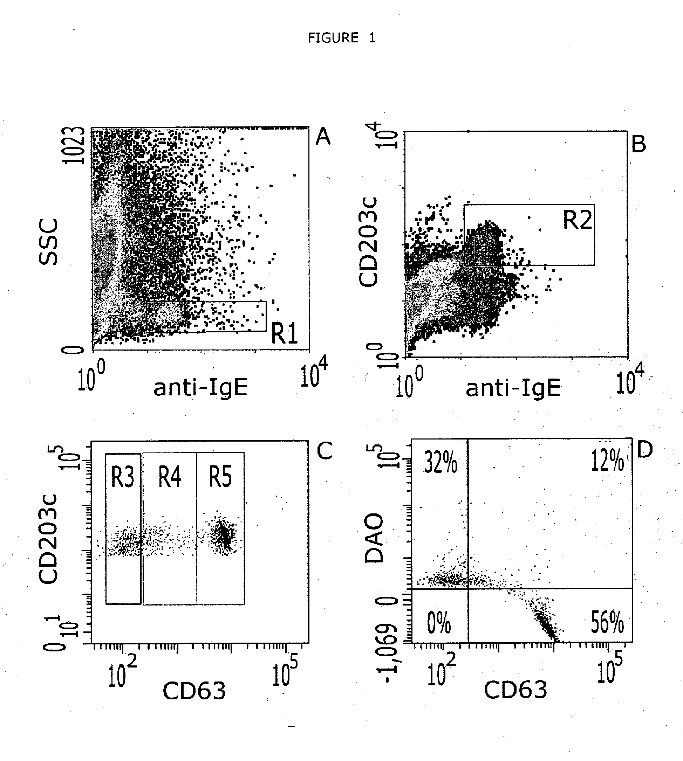

**Figure 1** provides the identification of resting and activated basophils following challenge with anti-IgE antibody using the method according to the present invention. Flow cytometric analysis using a density plot of side scatter (SSC) and anti-IgE (A). At least 1000 anti-IgE-Alexa Fluor 405 and anti-CD203c-APC positive basophils are gated out in a rectangular region (B). Within this gate co-expression of CD203c and CD63 was analyzed and revealed 3 subpopulations of CD63 expression: CD203c^{bright}CD63⁻ (R3), CD203c^{bright+}CD63^{dim+} (R4) and CD203c^{bright+}CD63^{bright+} (R5) (C). Histamine-containing basophils were defined as PE-DAO⁺ cells. Fifty-six percent of the basophils completely released their histamine content (DAO- cells) and are situated in lower-right quadrant of panel D. Note that the Y-axis in panel C and D is bi-exponential..

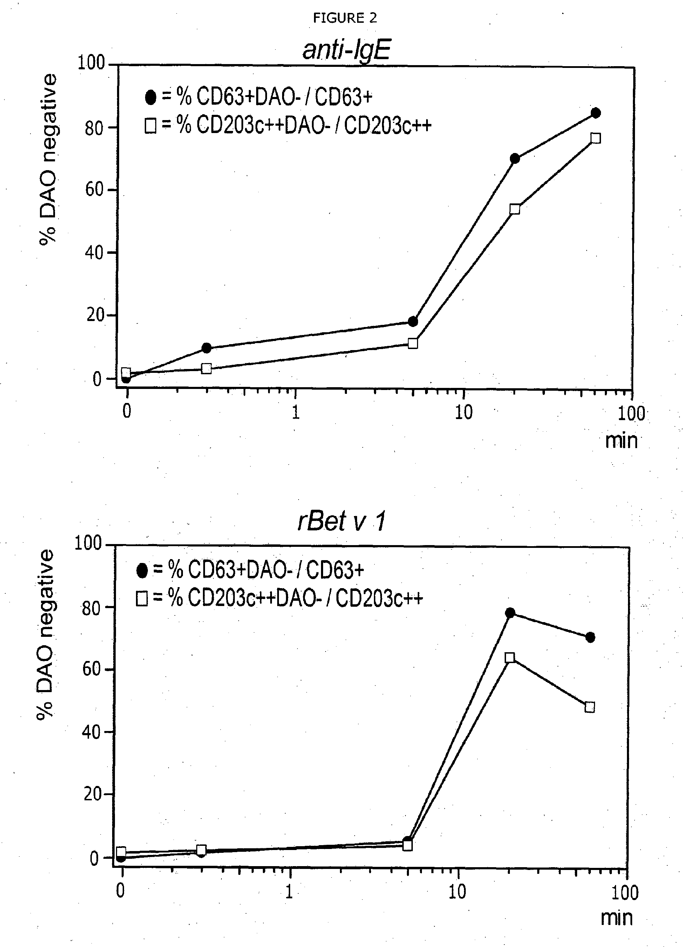

**Figure 2** provides the kinetics of histamine release in basophils after FCεRI cross-linking by anti-IgE antibody (top) or rBet v 1 allergen (bottom). Histamine release is expressed as % DAO⁻ cells by all CD203c^{bright} cells (%CD203c^{bright+}DAO-/CD203c^{bright+}) and by CD203c^{bright+} cells that co-express CD63 (% CD63+DAO⁻/CD63⁺).

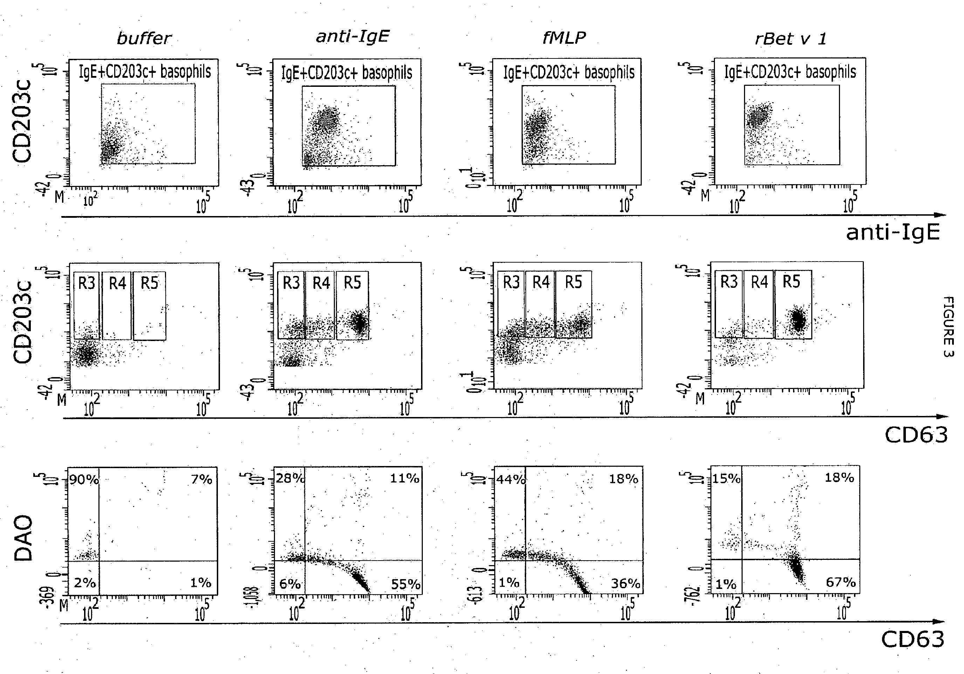

**Figure 3** provides the activation patterns of basophils after FcεRI cross-linking by anti-IgE antibody or rBet v 1 allergen or after stimulation with fMLP. Selection of anti-IgE-Alexa fluor 405 and anti-cD203c-APC positive basophils (upper panel), expression of the activation markers CD63 and CD203c (middle panel) and histamine release coupled to expression of CD63 (bottom panel) in cells incubated with (activation) buffer, anti-IgE antibody, fMLP and recombinant birch pollen allergen (rBet v 1). Basophils that completely released histamine (DAO- cells) are situated in lower-right quadrants of the bottom panel. Note that the Y-axis is bi-exponential. As in Figure 1, remark the distribution of activated basophils with a CD203c^{bright+}CD63⁻, CD203c^{bright+}CD63^{dim+} and CD203c^{bright+}CD63^{bright+} subpopulation. Upon stimulation with rBet v 1, 67% of the basophils completely released their histamine content.

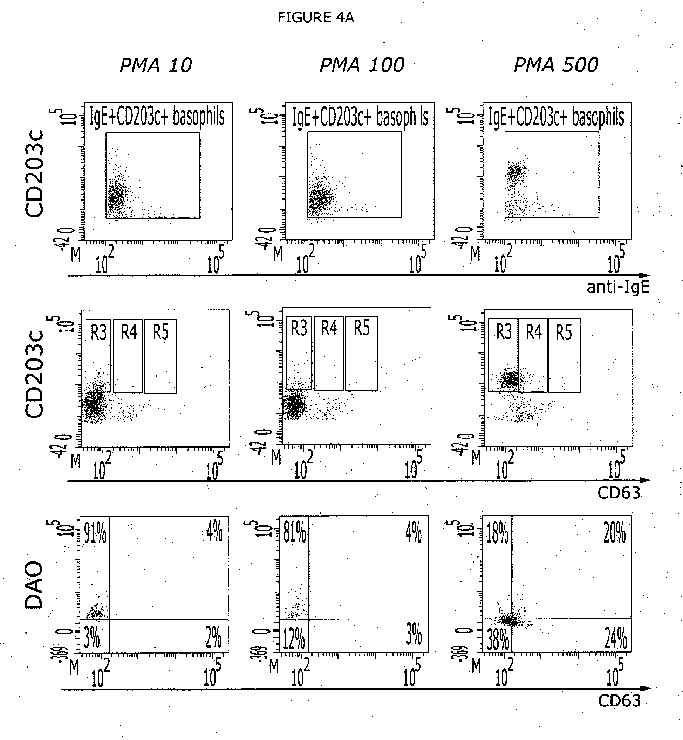

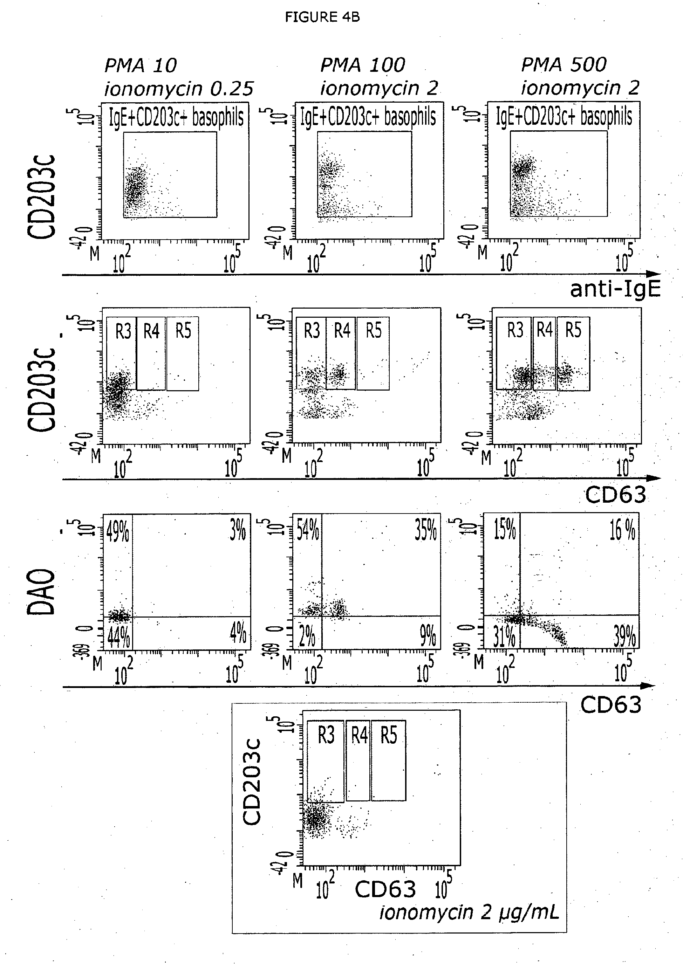

**Figure 4** provides activation patterns of basophils following stimulation with PMA without or with IO
   Selection of anti-IgE-Alexa fluor 405 and anti-cD203c-APC positive basophils (upper panel), expression of the activation markers CD63 and CD203c (middle panel) and histamine release coupled to expression of CD63 (bottom panel) in cells incubated with PMA 10, 100 and 500 ng/mL and PMA-ionomycin (IO) 10 ng/mL-0.25 µg/mL, 100 ng/mL-2 µg/mL and 500 ng/mL-2 µg/mL. Also shown is the expression of the activation markers CD63 and CD203c in cells incubated with ionomycin 2 µg/mL. Basophils that completely released histamine (DAO- cells) are situated in lower-right quadrants of the bottom panel. Note that the Y-axis is bi-exponential. As in Figure 1, remark the distribution of activated basophils with a CD203c^{bright+}CD63⁻, CD203c^{bright+}CD63^{dim+} and CD203c^{bright+}CD63^{bright+} subpopulation.

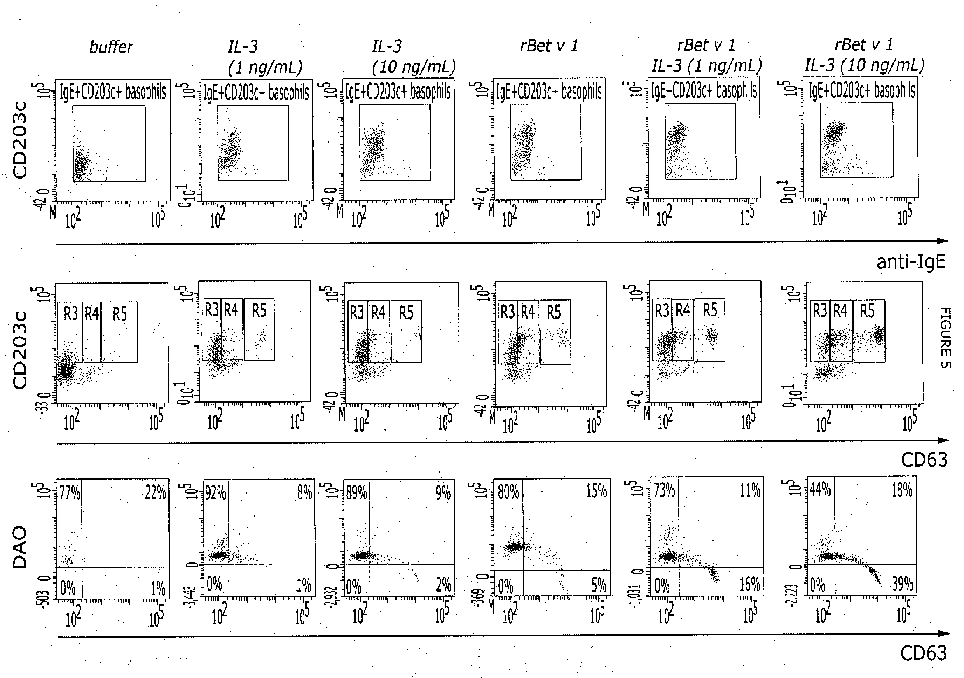

**Figure 5** provides activation patterns of basophils following incubation with IL-3 Selection of anti-IgE-Alexa fluor 405 and anti-cD203c-APC positive basophils (upper panel), expression of the activation markers CD63 and CD203c (middle panel) and histamine release coupled to expression of CD63 (bottom panel) in cells incubated with (activation) buffer, IL-3 1 ng/mL, IL-3 10 ng/mL, rBet v 1 0.01 µg/mL, IL-3 1 ng/mL + rBet v 1 0.01 µg/mL and IL-3 10 ng/mL + rBet v 1 0.01 µg/mL. Basophils that completely released histamine (DAO⁻ cells) are situated in lower-right quadrants of the bottom panel. Note that the Y-axis is bi-exponential. As in Figure 1, remark the distribution of activated basophils with a CD203c^{bright+}CD63⁻, CD203c^{bright+}CD63^{dim+} and CD203c^{bright+}CD63^{bright+} subpopulation.
**Figure 6** provides activation patterns of basophils further to stimulation with cephazolin sodium (Cefacidal®) in a patient showing an allergic reaction to cephazolin.
**Figure 7** provides activation patterns of basophils further to stimulation with pholcodine in a patient showing an allergic reaction to pholcodine.
**Figure 8** illustrates the histamine content of cells of an allergic patient after stimulation with wasp venom. The figure provides the median histamine content per cell as measured in a sample of a patient reacting to wasp venom prior to immunization (day 0) and 3 days and 6 months after immunization. The histamine content in a control sample is also provided.
**Figure 9** illustrates the decrease of histamine release over time as a result of venom immunotherapy.

Each of these illustrations represents particular embodiments of the features concerned and the corresponding features are not to be interpreted as limited to this specific embodiment.

### DETAI LED DESCRI PTI ON OF THE I NVENTI ON

The terminology used herein is not intended to be limiting, since the scope of the present invention will be limited only by the appended claims.
As used herein, the singular forms "a", "an", and "the" include both singular and plural referents unless the context clearly dictates otherwise.
The terms "comprising", "comprises" and "comprised of" as used herein are synonymous with "including", "includes" or "containing", "contains", and are inclusive or open-ended and do not exclude additional, non-recited members, elements or method steps. Where embodiments are referred to as "comprising" particular features, elements or steps, this is intended to specifically include embodiments which consist of the listed features, elements or steps.
The recitation of numerical ranges by endpoints includes all numbers and fractions subsumed within the respective ranges, as well as the recited endpoints.
The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.
Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.
Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.
The present invention is directed to cytometric analysis methods for the quantification of intracellular histamine and histamine release in mast cells and basophils. More particularly the methods of the invention allow the quantification of intracellular histamine and histamine release in individual mast cells and basophils. The basis of the invention is the finding that intracellular histamine can be qualitatively and quantitatively detected by cytometry by an enzyme-affinity method wherein histaminase diamine oxidase (DAO) is coupled to laser-excitable fluorochromes.
Mast cells and basophils are characterized by the presence of granules rich in histamine. Activation of mast cells and basophils ultimately leads to degranulation and the release of histamine. However, it is the histamine release as such which is physiologically relevant. Histamine release has traditionally been measured in the supernatant of activated mast cells and basophils, representing only an average of cells analyzed and not informing on cells or cell subpopulations with heterogeneity in responses. The present method allows localizing histamine at single cell level, which is an improvement over traditional methods. The methods of the present invention further allow the identification of activated basophils and mast cells.
The methods of the present invention require that the cells are adequately treated to allow the histaminase diamine oxidase to enter the cell and reach its substrate histamine. Thus, the methods of the present invention comprise a fixing step with a fixation buffer comprising a fixation reagent and a permeabilization step with a permeabilization reagent.
Methods of fixation are well known in the art. With "fixation buffer" is meant herein an aqueous buffered solution comprising a fixation reagent. Methanol- or (para)formaldehyde-based fixation reagents are preferred, more preferably (para)formaldehyde-based fixation reagents.
Optionally, the fixation buffer may further comprise a lysing reagent, most particularly a lysing reagent for red blood cells. The terms "lysis" and "lysing" are used interchangeably herein and relate to the process of lysing non nucleated blood cells (i.e. red blood cells) in order to avoid any interference with the cytometric analysis of nucleated blood cells. Accordingly, suitable lysing reagents are preferably selected from any kind of erythrocyte lysing reagents known from prior art. The use of a fixation buffer comprising a fixation reagent and a lysing reagent is particularly envisaged when a sample comprising red blood cells, such as a whole blood sample is analyzed. A fixation buffer comprising a fixation reagent and a lysing reagent advantageously allows lysing of red blood cells and fixing of leukocytes to be performed in a single step. An example of such fixation buffer comprising a fixation reagent and a lysing reagent is Phosflow Lyse/Fix Buffer (BD Biosciences) which is an aqueous buffered solution containing formaldehyde as a fixation reagent. However, it will be understood to the skilled person that the fixation and the lysing reagent may also be used separately.
In order for the histaminase diamine oxidase to gain access to intracellular histamine, the cells require permeabilization. Ideally, the permeabilizing reagent creates apertures in the cell membranes without affecting the gross morphology of the cell such that flow cytometric light scattering characteristics of the cell are not affected. Such methods of permeabilizing cells are well known in the art. Saponin- and Triton-X-100-based permeabilizing reagents are preferred, more preferably Triton-X-100-based permeabilizing reagents. With "permeabilization buffer" is meant herein an aqueous buffered solution comprising a permeabilization reagent.
In particular embodiments of the methods of the present invention, the mast cells and basophils are fixed with a fixation buffer comprising formaldehyde and permeabilized with a Triton-X-100 comprising permeabilizing buffer, more preferably a permeabilizing buffer comprising from 0.01 to 1 %, preferably from 0.02 to 0.5 %, more preferably from 0.05 to 0.2 %, most preferably about 0.1 % Triton-X-100.
In further particular embodiments of the methods of the present invention, the cells comprised in a whole blood sample are mixed with a fixation buffer comprising a lysing reagent and formaldehyde, such that red blood cells in the sample are lysed and the remaining cells are fixed, and the fixed cells are permeabilized with a Triton-X-100 comprising permeabilizing buffer, more particularly a permeabilizing buffer comprising from 0.01 to 1 %, preferably from 0.02 to 0.5 %, more preferably from 0.05 to 0.2 %, most preferably about 0.1 % Triton-X-100.
After fixing and permeabilization of the cells, the methods of the present invention further comprise the step of staining said mast cells and basophils intracellularly with fluorochrome-labeled histaminase diamine oxidase. The histaminase diamine oxidase is conjugated to a fluorochrome by techniques well known in the art. Different fluorochromes can be applied to conjugate the histaminase diamine oxidase such as, for example, fluorescent nanoparticles such as those sold under the trade name Alexa Fluor® Dyes, biotin-streptavidin labeled with phycoerythrin/Cy7, fluorescein isothiocyanate (FITC), Horizon-V500, phycoerythrin. In a particular embodiment, the histaminase diamine oxidase is conjugated to phycoerythrin (PE), which has been shown to give a good resolution. In further particular embodiments, the histamine diamine oxidase is conjugated to Horizon V500 (BD Biosciences). This allows wider application of the technology as several surface molecules that can be quantified simultaneously with histamine release are traditionally coupled to PE.
Staining with said fluorochrome-labeled histaminase diamine oxidase may be achieved through incubating said fixed and permeabilized mast cells and basophils with said said fluorochrome-labeled histaminase diamine oxidase. This is of particular interest when combining histamine-DAO staining with intracellular CD63 staining (available in the granules of non-activated cells). It has been observed by the present inventors that this results in a further improvement of the quantification of intracellular histamine. Indeed, reproducibility of histamine quantification resulted in mean variation coefficient of 10 % (95CI: 2-17) number CD63% positive cells and mean variation coefficient of 40 (95CI: 7-72) mean histamine content per cell.

In particular embodiments, the staining is performed at 37°C for a predetermined period of time, such as for example for up to 1 h, preferably for between 30 min and 1 h, more preferably for about 45 min.
Thus, the present invention provides methods for quantifying intracellular histamine and histamine release in individual mast cells and basophils comprising the steps of:
a) fixing said mast cells and basophils with fixation buffer comprising a fixation reagent;
b) permeabilizing said mast cells and basophils with permeabilization reagent;
c) staining said mast cells and basophils intracellularly with fluorochrome-labeled histaminase diamine oxidase; and
d) analyzing said stained mast cells and basophils on a cytometer, wherein the signal from said fluorochrome-labeled histaminase diamine oxidase is indicative of the quantity of histamine present per cell in each of said mast cells and basophils.
In particular embodiments, as described above, the staining with fluorochrome-labeled histaminase diamine oxidase is combined with other markers. Such markers can include "selection markers", "signaling molecules", "activation markers" and/or "inhibitory receptors".
In particular embodiments of the methods of the present invention, the mast cells and basophils are identified and/or selected out by way of one or more selection markers.
As used herein, a "selection marker", refers to any molecule that may be used, alone or in combination, to facilitate the identification of mast cells and basophils. These selection markers are typically cellular membrane molecules, although intracellular molecules may also be used. Suitable selection markers include, but are not restricted to, IgE, FcεRI, the lineage-specific CD203c, CD123/HLADR and chemokine receptor 3 (CCR3). The one or more selection markers referred to herein may include activation markers, as will be detailed below. Typically the selection or activation markers, inhibitory receptors or signalling molecule are detected by way of an antibody directed against the marker. The term antibody as used herein is meant to include any antigen-binding fragment which can be used in the context of the present invention. Antibodies directed against the markers envisaged in the context of the present invention are known to the skilled person and are commercially available. Thus, in particular embodiments, the invention provides methods which comprise detecting a basophil and/or mast cell selection marker with an antibody directed against a mast cell and/or basophil selection marker.
In further particular embodiments of the methods of the present invention the antibodies directed against the selection markers used in the context of the present invention are labeled, most particularly fluorochrome labeled.
Staining said mast cells and basophils with said fluorochrome-labeled antibody directed against a mast cell and/or basophil selection marker may be performed according to well known techniques for staining cell surface molecules, such as, for example, incubating said mast cells and basophils with said antibody on ice for a predetermined period of time, such as for example for up to 1 h, preferably for between 10 min and 30 min, more preferably for about 20 min.
The antibodies used throughout the present disclosure may be labeled with any fluorochrome known in the art. Non-limiting examples of fluorochromes that may be used include fluorescein isothiocyanate (FITC), Texas Red (sulforhodamine isothiocyanate), allophycocyanin (APC), Cy3, Cy5, Oregon Green isothiocyanta, phycoerythrin (PE), fluorescent nanoparticles such as those sold under the trade name Invitrogen Q-dots® or Dyomics dyes such as DY-647 or DY-747. When using multiple fluorochromes in the methods of the invention, said fluorochromes preferably have distinct emission spectra, more preferably they are separated spectrally from one another.

Typically, the mast cells and basophils are stained for the selection marker prior to the fixing step and staining with the histaminase. Thus, in particular embodiments, methods for quantifying intracellular histamine and histamine release in individual mast cells and/or basophils comprise the steps of:
a) staining said cells for the presence of one or more selection markers for basophils and/or mast cells
b) fixing said mast cells and/or basophils with fixation buffer comprising a fixation reagent;
c) permeabilizing said mast cells and/or basophils with permeabilization reagent;
d) staining said mast cells and/or basophils intracellularly with fluorochrome-labeled histaminase diamine oxidase; and
e) analyzing said stained mast cells and/or basophils on a cytometer, wherein the signal from said fluorochrome-labeled histaminase diamine oxidase is indicative of the quantity of histamine present per cell in each of said mast cells and basophils.
The mast cells and/or basophils to be analyzed using the methods of the present invention may be comprised in a blood sample, e.g. a whole blood sample or a (partially) purified blood sample, a tissue biopsy, e.g. a skin biopsy, or any other source which comprise multiple mast cells and/or basophils. The mast cells and basophils may also be provided as an isolated (pure) population of mast cells and/or basophils. In a preferred embodiment, a whole blood sample is analyzed. Basophils represent a small white blood cell subpopulation. Thus, in particular embodiments, basophils are comprised in a white blood cell fraction. Mast cells are found resident in tissues throughout the body, particularly in association with structures such as blood vessels and nerves, and in proximity to surfaces that interface the external environment.
When analyzing a blood sample, the methods of the present invention may comprise the step of treating the sample with EDTA, heparin or any other anticoagulants in order to prevent coagulation.
When analyzing a sample potentially comprising red blood cells, the methods of the invention may comprise the step of lysing the red blood cells with a lysing reagent. In particular embodiments the lysing reagent is present in the fixation buffer. In alternative embodiments, the methods of the invention comprise a separate step, wherein the sample is contacted with a lysing buffer comprising a lysing reagent. Examples of lysing reagents are provided herein below.
The methods of the present invention further comprise the step of analyzing said stained mast cells and basophils on a cytometer. The analysis by cytometry is carried out as known from the prior art. Cytometry allows the rapid measurement of light scatter or morphometry and fluorescence emission produced by suitably illuminated cells or particles. The cells or particles produce signals when they individually pass through or are exposed to a beam of light. Each cell or particle is measured separately and the output represents cumulative individual cytometric characteristics. An important analytical feature of modern cytometers is their ability to measure multiple cellular parameters. The scattered light and fluorescence emissions of each cell or particle are collected by detectors and sent to a computer, where the distribution of the population with respect to the different parameters is represented. Scattered light collected in the same direction as the incident light is related to cell size, and scattered light collected at an angle of 90 indicates particle complexity. Morphometry and particle complexity is related to cell surface characteristics and the number and type of organelles present in the cell. Size and complexity are considered intrinsic parameters since they can be obtained without having to stain the sample. To obtain additional or more specific information, samples may be stained using different fluorochromes. For instance, said fluorochromes may be conjugated to antibodies which specifically bind to antigens to gain information on said antigens. In the present invention the enzyme histaminase diamine oxidase is conjugated to a fluorochrome in order to obtain information on its substrate histamine.
Using cytometry, a specific population of cells can be "gated" and analyzed separately from the remainder of the population. For example, mast cells and basophils may be gated out from a whole blood sample by applying side scatter and staining of a mast cell and basophil selection marker.
Fluorescence activated flow cytometry provides a method for sorting a heterogeneous mixture of biological cells into two or more containers, one cell at a time, based upon the specific light scattering and fluorescent characteristics of each cell. In laser scanning cytometry (LSC) a scan line oscillates on the cell and the fluorescent energy emitted by each cell is collected and specifically detected. This allows the analysis of samples on different substrates.
Cytometers useful for carrying out the present invention include conventional and state of the art flow cytometers made by Beckman Coulter, Toa Medical Electronics, Partec, Bio-Rad, or Becton Dickinson and image cytometers made by General Electric, and Becton Dickinson.
In particular embodiments of the methods of the present invention, the signals generated from said fluorochrome-labeled histaminase diamine oxidase may be compared to a reference value. In particular embodiments, the reference value is indicative of degranulation of mast cells and basophils and is used to determine whether a mast cell or basophil has undergone degranulation or not. A reference value may provide the signal from a mast cell or basophil that has undergone degranulation, for instance a mast cell or basophil that has been incubated with an activator. Additionally or alternatively, the a resting basophil or mast cell may be used as a reference value.
To ensure calibration of the intracellular histamine detection, calibration particles may be added to the mast cells and basophils to be analyzed. With "calibration particles" are meant herein a set of fluorescently labeled particles which can be used to calibrate the detection with the fluorochrome-labeled histaminase. Most particularly, the particles are labeled with one or more fluorochrome. In further particular embodiments, the fluorochrome is identical to that of the fluorochrome-labeled histaminase. In further particular embodiments, the particles are labeled with a mixture of fluorochromes at different intensities that allows excitation at any wavelength between 365 and 650 nm that enable to construct a calibration curve in the channels of the flow cytometer in which the fluorochrome-labeled histaminase diamine oxidase is measured. A suitable example of calibration particles include SPHERO^{®} Ultra Rainbow particles (Spherotech, Lake Forest, IL, US). A further example of suitable calibration particles are the QuantiBrite^{®} particles. Indeed, the different quantified levels of R-phycoerythrin (PE) intensity on the separate beads provide for an exact quantification of the available PE molecules inside the individual cells that are stained with the PE-labeled diamino-oxidase.
In particular embodiments, a calibration curve is made using the calibration particles based on the strongest staining cell. Typically this is a resting cell, as this cell retains all of its histamine. Resting cells can be identified based on the expression of selection markers but no, or only limited expression of activation markers. For instance, resting basophils express CCR3 but not CD63. The amount of histamine in the activated cells can then be quantified based on the calibration curve. As the amount of histamine in a resting cell may vary from one individual to another, it may be of interest to calibrate detection for every patient. Thus, in particular embodiments, the methods of the invention will comprise calibrating the histamine content using calibration particles and/or based on the histamine content of a resting cell.
In particular embodiments the methods of the present invention comprise analyzing the signal generated at different time points. This allows to monitor the evolution of histamine content in the cells, and thus allows a measurement of histamine release by the cells at different time points, i.e. same samples or parallel samples incubated for longer time periods etc..
The methods of the invention may be used to further characterize activated mast cells and basophils, more particularly to determine the relationship between the expression of activation markers and the histamine release in these cells. This can be achieved by staining the cells for use in the methods of the present invention with an activation marker.
Thus, in further embodiments, the methods according to the present invention further comprise the step of staining said mast cells and/or basophils for the presence of an activation marker.
As used herein, an "activation marker" refers to a molecule that may be used to identify activated mast cells and basophils. As used herein, "activated mast cells and basophils" denote mast cells and basophils that have undergone cross-linking of FcεRI-bound IgE antibodies. Activated basophils not only release potent mediators such as histamine but also up-regulate the expression of different membrane markers, e.g., CD63, CD203c, CD11b, CD13, CD107a, CD164, CD300a. Accordingly, these molecules may be used as activation markers for basophils.
The same principles are applicable for mast cells but using other activation markers such as CD107 and CD117 (c-kit). As indicated above, the expression of markers is typically identified using labeled antibodies directed against these markers. In particular embodiments the antibodies are fluorochrome labeled (examples of fluorochrome labels are provided above). The staining of mast cells and basophils with a fluorochrome-labeled antibody directed against an activation marker may be performed according to well known techniques for staining cell surface molecules, such as, for example, incubating said mast cells and basophils with said antibody on ice for a predetermined period of time, such as for example for up to 1 h, preferably for between 10 min and 30 min, more preferably for about 20 min.
The dual labeling of intracellular histamine and intracellular and/or membrane activation markers is advantageous in that it allows analyzing the relationship between expression of activation markers and histamine release, which is still elusive at present.
In particular embodiments it can be of interest to determine the reaction of mast cells and/or basophils to an activator. For instance, it may be of interest to determine whether cells are capable of reacting normally to an activator. Alternatively, in the context of screening compounds for the effect on histamine content and/or release, it may be of interest to add an activator to the cells. Thus, in particular embodiments, the present invention provides methods wherein the mast cells and/or basophils are incubated with an activator prior to analyzing said mast cells and basophils according to the methods disclosed herein.
An "activator" refers herein to any substance that induces activation of mast cells and basophils, thereby leading to degranulation and histamine release. Non-limiting examples of activators include anti-IgE antibody, anti-FcεRI antibody, N-formyl-L-methionyl-L-leucyl-L-phenylalanine (fMLP), phorbol 12-myristate 13-acetate/ionomycin (PMA/ionomycin), complement factor 5a (C5a), platelet activation factor (PAF), toll-like receptor (TLR) agonists, ionomycin, calcium ionophore A23187, lipopolysaccharides (LPS) and phytohaemagglutinins (PHA). When using mast cells and basophils from an allergic patient, natural or recombinant allergen and components thereof causing an allergic reaction in said patient may also be used as activator. Preferably, the activator is selected from the group comprising: anti-IgE antibody, N-formyl-met-leu-phe (fMLP) and phorbol 12-myristate 13-acetate/ionomycin (PMA/ionomycin) or allergen.
As detailed above, the methods of the present invention may be used to identify the effect of a test compound on the histamine content and/or release of a mast and/or basophil sample. Thus, in particular embodiments, the present invention provides methods wherein the mast cells and/or basophils are incubated with a test compound prior to analyzing said mast cells and basophils according to the methods disclosed herein.
The nature of the test compound is not critical and includes but is not limited to a protein, a peptide or a mixture of proteins or peptides or a protein extract from a biological source, preferably selected from the group of insect venoms, foods (fruits, vegetables, seeds, legumes, nuts, spices, fish, shellfish, mollusks, egg, fowl, meat and milk), tree pollens, grass pollens, weed pollens, epidermal and animal proteins, dust and storage mites, insects, parasites, microorganisms, small molecules, DNA, RNA etc.
Typically, the methods of the invention will include the necessary positive and/or negative controls, such as but not limited to samples to which no test compound has been added, samples to which a known activator has been added etc, samples to which a control compound has been added etc.. Suitable controls are known to the skilled person.
The incubation of mast cells and/or basophils with an activator or a test compound is preferably performed at optimal temperature conditions, preferably in the range of from 30 to 40 °C, more preferably at about 37°C. The time of incubation may range from between 10 seconds up to 2 hours, preferably from between 20 seconds up to 100 min, more preferably from between 10 min up to 80 min, even more preferably from between 20 min up to 60 min. In particular embodiments of the methods of the invention, the incubation is performed at different time points so as to allow a definition of histamine release under gradual, piecemeal release or sudden and complete release. The incubation is preferably stopped by chilling on ice and adding a stop solution containing 1 to 20 mmol/l of EDTA, preferably 5 to 15 mmol/l, more preferably 10 mmol/l.
The incubation of mast cells and basophils with an activator or a test compound is preferably performed in an activation buffer. A suitable activation buffer may contain optimum calcium and magnesium concentration in optimized physico-chemical conditions. A suitable example of activation buffer is Hank's balanced salt solution that additionally contains 20 µM HEPES and 7.5% NaHCO₃ at pH 7.4. The activation buffer may also be used for deleting the activator and the test substance.
In particular embodiments the activation buffer may comprise interleukin(IL)-3. The inventors have found that priming the mast cells and basophils with IL-3 potentiates the effect of incubation with an activator, i.e. will promote degranulation, while IL-3 itself does not induce degranulation.
As indicated above, the signal of the fluorochrome-labeled histaminase diamine oxidase of mast cells and basophils that have been incubated with an activator prior to analysis according to the methods of the present invention may be used as a reference value which is indicative of degranulation of mast cells and basophils.
The signal of the fluorochrome-labeled histaminase diamine oxidase of mast cells and basophils that have been incubated with a test compound according to the present method may be compared to a reference value indicative of degranulation of mast cells and basophils. A signal lower than said reference value indicates that the test compound has induced degranulation and is indicative of an allergenic effect of said test compound, whereas a signal higher than said reference value indicates that the test compound has not induced degranulation.

In another aspect, the present invention provides kits for quantifying intracellular histamine and histamine release in individual mast cells and basophils. The kits of the present invention are particularly suited for carrying out the methods of the present invention.
In particular embodiments, the present invention provides a kit for quantifying intracellular histamine and histamine release in individual mast cells and basophils comprising:
- a fluorochrome-labeled histaminase diamine oxidase;
- a fluorochrome-labeled antibody directed against a selection marker;
- fixation buffer comprising a fixation reagent; and
- permeabilization buffer comprising a permeabilization reagent.
In a particular embodiment said fixation reagent comprises (para)formaldehyde or methanol, more preferably (para)formaldehyde. In a further embodiment said permeabilization reagent comprises Triton-X-100 or saponin, more preferably Triton-X-100.
The kits of the invention comprise a fluoro-chrome labeled antibody against a selection marker. In particular embodiments said fluorochrome-labeled antibody directed against a selection marker is selected from fluorochrome-labeled antibody directed against a selection marker such as but not limited to IgE, FcεRI, the lineage-specific CD203c, CD123/HLADR and chemokine receptor 3 (CCR3).
As detailed above, the nature of the fluorochrome of the fluorochrome-labeled histaminase diamine oxidase is not critical to the present invention. However, in particular embodiments, the kits of the invention comprise phycoerythrin (PE)-labeled histaminase diamine oxidase.
Suitable fixation buffers and permeabilization buffers envisaged in the context of the present invention are described herein and further known to the person skilled in the art.
The kits of the present may comprise one or more additional reagents which are of particular use for carrying out the methods of the invention.
Thus, in particular embodiments, the kits additionally comprise a lysing reagent. The lysing reagent may be comprised in the fixation reagent or may be provided in a separate lysing buffer. Suitable lysing reagents include any kind of erythrocyte lysing buffers known from the art. A suitable fixation buffer comprising a fixation reagent and a lysing reagent is Phosflow Lyse/Fix Buffer (BD Biosciences) which is an aqueous buffered solution containing formaldehyde as a fixation reagent.
In further particular embodiments, the kit further comprises a reference value or tools for obtaining a reference value, such as a reference which is indicative of degranulation of mast cells and basophils. A reference value may be determined by incubating mast cells and/or basophils with an activator which induces degranulation. Accordingly, the kit may also comprise an activator and activation buffer. Suitable activators include, but are not restricted to, anti-IgE antibody, N-formyl-met-leu-phe (fMLP) and phorbol 12-myristate 13-acetate/ionomycin (PMA/ionomycin).
In particular embodiments, the kit may additionally comprise a positive control and/or a negative control for degranulation. Examples of suitable positive controls include an activator, while a suitable negative control is, for instance, activation buffer not comprising an activator.
In particular embodiments, the kit according to the invention comprises calibration particles. Examples of calibration particles are described herein above.
In particular embodiments, the kit comprises one or more fluorochrome-labeled antibodies directed against an activation marker. More particularly, the fluorochrome-labeled antibody directed against an activation marker is a fluorochrome-labeled antibody directed against CD63, CD203c, CD11b, CD13, CD107a, CD164 or CD300a. In particular embodiments, the kits of the invention comprise an anti-CD63 antibody or a fluorochrome-labeled anti-CD203c antibody.
In further embodiments, the kit further comprises instructions for use of the kit according to the methods of the present disclosure. In a particular embodiment, the kit further comprises instructions for quantifying the intracellular histamine content of individual mast cells and basophils.

Another aspect of the present invention relates to the applications of the methods and kits of the present invention.
As detailed above, the methods and the kits of the present invention may be useful for quantifying intracellular histamine and histamine release in individual mast cells and/or basophils.
Typically, the methods of the present invention are used to determine histamine content and/or release of basophils and mast cells in biological samples, more particularly human or animal samples. The determination of histamine content and/or release can be determined independently of whether the cell is considered as "resting" or "activated" based on activation markers.
In particular embodiments the methods and the kits are used for quantifying degranulation of mast cells and/or basophils. Activation of mast cells and basophils ultimately lead to degranulation and the release of histamine. Accordingly, in one embodiment the methods and the kits may be used for quantifying activated mast cells and basophils.
In further particular embodiments, the methods and the kits may be used for an *in vitro* allergy test, wherein mast cells and/or basophils are analyzed that are comprised in a sample from a patient, such as a (whole) blood sample or a (skin) biopsy. More particularly the methods and kits of the invention are of interest for diagnosing an allergy to drugs such as curarising neuromuscular blocking agents, antibiotics and narcotics (opiates and opioids). More particularly the invention is of interest in the context of diagnosing an allergy to narcotics, as current methods are unreliable.
In particular embodiments, the presence of degranulated mast cells and/or basophils in said sample is indicative of an allergic reaction in said patient. In further particular embodiments, the degree of histamine release in response to the allergen determined in a sample of the patient is indicative of an allergy to said allergen. Thus, these methods may comprise determining the histamine content in a sample prior to and after contacting with the allergen. In particular embodiments, the methods comprise comparing the histamine content of the cells with that of a control.
Thus in particular embodiments, the present invention provides in vitro diagnostic methods for determining and/or characterizing allergic conditions. More particularly, the methods and kits are envisaged for use in the diagnosis of all allergic conditions that rely upon IgE-mediated as well as IgE-independent activation of mast cells and basophils. More particularly, the methods and kits of the invention will allow to distinguish between a piecemeal activation and an anaphylactic reaction of the basophils and mast cells.

Similarly the methods and kits of the invention can be used to monitor the effect of drugs that aim to silence mast cells and basophils (e.g. in mastocytosis) on patients, by analysis of patient samples. This includes monitoring of allergen-specific immunotherapy and anti-IgE treatment (e.g. omalizumab). Thus, in particular embodiments, the invention provides methods and kits are for monitoring efficacy of treatment, such as allergen-specific immunotherapy.
In particular embodiments, the methods of the present invention are used to monitor the effect of allergen-specific immunotherapy. Indeed, the present invention not only makes it possible to determine the histamine content of basophils in patient samples prior to challenging them but allows the close monitoring of the effect of such a therapy on the histamine content of basophils of the patient. Examples of allergies where immunotherapy has been found effective include, but are not limited to pollen, dust, and animal dander allergies. In particular embodiments of the invention, the methods are used to determine the effectiveness of immunotherapy with venoms such as wasp, bee and bumblebee venoms.
Thus, in particular embodiments, the methods of the invention are used to evaluate the effect of immunotherapy with hymenoptera venom, more particularly on the basophil responsiveness. In particular embodiments, the invention provides methods for determining the effect of immunotherapy with an allergen on a patient, the method comprising determining whether there is a decrease in the intracellular histamine content in resting cells in said patient. In particular embodiments, these methods comprise comparing the intracellular histamine content of resting cells in a sample of said patient prior to treatment with said allergen to the intracellular histamine content of resting cells after said treatment.

In particular embodiments, the methods and kits are used to determine the allergenic or anti-allergenic effect of a compound.
Typically, this encompasses testing a sample with and without the test compound and comparing the histamine content and/or release in the basophils and/or mast cells of the sample. In a further embodiment, the methods and the kits may be used for high-throughput screening of compounds, more particularly the high throughput screening of the allergenic effect of compounds or the effect of drugs on an induced allergenic effect. In further embodiments the methods and kits may be used for quantifying the histamine content of individual mast cells and basophils. More particularly this can be of use in the study of intracellular signaling (Ebo, 2007, Clin Exp All, 37:1668-75)
In one embodiment, the methods and kits may be used for studying the histamine release pattern induced by a compound, such as an activator or a test compound, wherein the mast cells and basophils are incubated with said compound at different time points. Histamine release may be defined as gradual piecemeal release or sudden and complete release.
In a further embodiment, the methods and kits may be used for studying the relationship between histamine release and expression of activation markers.

The inventors have further found that in patients who are allergic to specific allergens, basal histamine content per cell (in resting basophils that are not activated with the allergen) is significantly higher as compared to control individuals which have been contacted with the allergen. This is indicative of a difference in histamine metabolism between patients and control individuals. Thus, patients having an allergy against an allergen would benefit from treatment with compositions which can reduce histamine accumulation in the cells. More particularly drugs targeting histidine decarboxylase (involved in synthesis of histamine) are envisaged to be beneficial for such patients.
In this regard the methods of the present invention provide an easy in vitro model to assess the effect of these histidine decarboxylase inhibitors. Accordingly, a further aspect of the present invention relates to the use of the methods and tools described herein to assess the therapeutic potential of drugs targeting histidine decarboxylase for patients who are allergic to specific allergens. In particular embodiments such methods involve determining with the methods of the present invention for a particular patient whether the drug is capable of reducing the basal histamine content of basophils in said patient. Examples of known histidine decarboxylase inhibitors are catechin, tritoqualine an atypical antihistaminic (Hypostamin©). Similarly, the methods can be used to screen compounds for their potential therapeutic activity in patients who are allergic to one or more allergens.

The inventors have further found that human basophils express the inhibitory receptor CD300a that suppresses IgE-dependent activation of these cells as seen during life-threatening anaphylaxis (Sabato et al. Clin Cytom B, 2012). Thus, this CD300a can be used as a target for anti-allergenic treatment. The methods of the present invention thus provide a reliable tool to analyze the effect of engagement of inhibitory receptors such as CD300a, CD200R, Siglec-8 on histamine synthesis and release by individual basophils and mast cells. Accordingly, a further aspect of the present invention relates to methods for identifying drugs with anti-allergenic potential, which methods comprise determining the binding of said compound to CD300a and further determining whether said compound can affect histamine synthesis or release by basophils. In particular embodiments, the effect of the compounds on histamine synthesis and/or release by basophils is determined by the methods described herein.

### EXAMPLES

In the following examples 1 to 5 endotoxin-free heparinized whole blood samples from healthy control individuals and birch pollen allergic patients were used. Birch pollen allergic patients were selected upon clinical history documented by standardized skin tests and quantification of sIgE.

### Example 1

The present example describes the identification of resting and activated basophils following challenge with anti-IgE antibody using the method according to the present invention.
Aliquots of 200 µL whole blood from patients and control individuals were challenged at 37 °C with 200 µL anti-IgE antibody (10 µg/mL; clone G7-18, BD Biosciences, Erembodegem, Belgium) for 20 min. The reaction was stopped by chilling on ice, adding 1 mL ice-cooled PBS/10 mmol/L EDTA and centrifugation for 5 min (4 °C, 200xg).
The cells were then stained with 20 µL of monoclonal anti-human IgE (clone GE-1, Sigma Aldrich GmBH, Steinheim, Germany) labeled with Alexa Fluor 405 (Molecular Probes, Invitrogen, Paisley, UK) as described by the manufacturer and 10 µL of monoclonal anti-human CD63-FITC (clone H5C6, BD Biosciences) and 10 µL CD203c-APC (clone NP4D6 Biolegend, San Diego, CA, USA) for 20 min on ice.
The cells were subsequently lysed/fixed with 2 mL Lyse/Fix Buffer (BD Biosciences) for 20 min at 37 °C. Cells were washed with and re-suspended in PBS with 0.1 % Triton-X-100 (PBS-TX, pH=7.4).
To stain intracellular histamine 10 µL Phycoerythrin-labeled (labeling was performed by BD Biosciences) diamine oxidase (DAO, Sigma-Aldrich) was added and incubated at 37 °C for 45 min. Cells were washed with 0.3 mL PBS, re-suspended in PBS with 0.1% sodiumazide.
Flow cytometric analysis and quantification of basophil activation was performed on a FACSCanto II flow cytometer (BD Immunocytometry Systems, San Jose, CA, USA). Standardization of intracellular histamine content was performed using standardized fluorospheres (SPHERO Ultra Rainbow Calibration particles, Spherotech, Lake Forest, IL, USA) as described by the manufacturer. Results were expressed as mean fluorescence intensity (MFI) and used where appropriate.

Side scatter and anti-IgE staining were applied to gate out at least 1000 basophils (Figure 1A, R1). Within this gate activated basophils were identified as CD203c^{bright+} (Figure 1B, R2). In these CD203c^{bright+} cells, 3 types of CD63 expression were delineable: i) a CD63- subpopulation, ii) an intermediate CD63^{dim+} subpopulation and finally, iii) a CD63^{bright+} subpopulation (Figure 1C, respectively gates R3, R4 and R5). Histamine-containing basophils were defined as DAO⁺ cells (Figure 1D). It emerged that histamine release in these anti-IgE stimulated basophils was generally restricted to the CD63^{bright+} cells.. Note that in this example of IgE-stimulated basophils 56% of basophils demonstrated complete degranulation (right lower quadrant of panel D).

This example shows that intracellular histamine and histamine release by *in vitro* activated basophils can successfully be analyzed using multicolor flow cytometry applying an enzyme-affinity-fluorochrome method based on the affinity of the histaminase diamine oxidase (DAO) for its substrate. Furthermore it shows that the combination of fixation with paraformaldehyde and permeabilization with Triton X-100 give good results for combined staining of intracellular histamine and the surface activation markers CD63 and CD203c.

### Example 2

The present examples describes the kinetics of activation marker expression and histamine release after FcεRI cross-linking by anti-IgE antibody and recombinant Bet v 1 (rBet v 1) allergen.
Aliquots of 200 µL whole blood from patients and control individuals were challenged at 37 °C with 200 µL anti-IgE antibody (10 µg/mL; clone G7-18, BD Bioscience) or recombinant Bet v 1 allergen (rBet v 1: 0.01 µg/mL, kindly provided by Stallergènes, Antony Cedex, France) at different time points (ranging from 20 seconds up to 60 minutes). The reactions were stopped by chilling on ice, adding 1 mL ice-cooled PBS/10 mmol/L EDTA and centrifugation for 5 min (4 °C, 200xg).
The cells were further processed and analyzed according to the protocol of Example 1.

Figure 2 shows that challenging the cells with (optimal concentrations of) anti-IgE and allergen elicits a quick histamine release that starts within minutes and peaks at about 15-20 minutes.

Kinetics of histamine release obtained by carrying out the flow cytometric method of the present invention are consistent to the findings with traditional techniques.

### Example 3

The present example shows the activation patterns of basophils following FcεRI cross-linking by anti-IgE antibody or recombinant Bet v 1 (rBet v 1) allergen or following challenge with fMLP.
Aliquots of 200 µL whole blood samples were challenged at 37 °C with 200 µL anti-IgE antibody (10 µg/mL; clone G7-18 BD Bioscience, Erembodegem Belgium) as positive control, 200 µL activation buffer (Hank's balanced salt solution (Invitrogen, Paisley, UK) with 20 µM HEPES and 7.5 % NaHCO₃, pH 7.4, without IL-3) as a negative control, N-formyl-met-leu-phe (fMLP: : 0.5 µg/mL) (Sigma-Aldrich, St-Louis, MO, SA) or recombinant Bet v 1 (rBet v 1: 0.01 µg/mL, kindly provided by Stallergènes, Antony Cedex, France) allergen for 20 min. The reactions were stopped by chilling on ice, adding 1 mL ice-cooled PBS/10 mmol/L EDTA and centrifugation for 5 min (4 °C, 200xg).
The cells were further processed and analyzed according to the protocol of Example 1.

Cross-linking of FcεRI by anti-IgE and rBet v 1 in birch pollen allergic patients and challenging cells with fMLP almost invariantly resulted in a significant up-regulation of CD203c expression (CD203c^{bright+}). In these CD203^{bright+} cells, the distribution of CD63 expression was bimodal, i.e. negative (CD63⁻) or positive (CD63⁺). However, in most cases the CD63⁺ cells comprised a CD63^{bright+} and CD63^{dim+} subpopulation (fig 3 mid panel, R4 and R5). CD203c^{bright+}CD63⁺ cells demonstrated a clear histamine release, particularly CD63^{bright+} cells (fig 3, bottom panel). In contrast, cCD203c^{bright+} cells that did not co-express CD63 upon stimulation did not release histamine (fig 3, bottom panel).

Cross-linking of FcεRI by anti-IgE or challenging with fMLP in healthy control individuals disclosed almost similar results (data not shown). Healthy control individuals demonstrated no up-regulation of CD203c or CD63 and did not release histamine upon challenging the cells with rBet v 1 (data not shown).

This example confirms that basophils that significantly up-regulate CD203c upon stimulation with anti-IgE antibody, allergen or fMLP, generally exhibit a bi-modal distribution of CD63 expression, i.e. a CD63 negative and positive subpopulation (Buhring et al, 2004, Int Arch Allergy Immunol, 133:317-329; Hennersdorf et al, 2005, Cell Res, 15:325-335; Chirumbolo et al, 2008, Clin Mol Allergy, 6:12). However, the individual cell responses of CD63 expression are generally quite heterogeneous, varying from completely absent (CD63⁻) over weak or modest (CD63^{dim+}) to very strong (CD63^{bright+}). This heterogeneity parallels the observations by others who demonstrated low and high CD63⁺ populations of varying sizes upon IgE-mediated stimulation of the cells (Buhring et al, 2004, Int Arch Allergy Immunol, 133:317-329; Hennersdorf et al, 2005, Cell Res, 15:325-335; Chirumbolo et al, 2008, Clin Mol Allergy, 6:12).

### Example 4

The present example shows the activation patterns of basophils following stimulation with PMA without or with IO.

Aliquots of 200 µL whole blood samples were challenged at 37 °C with phorbol 12-myristate 13-acetate (PMA; 500, 100 or 10 ng/mL) without or with ionomycin (IO; 0.25 or 2 µg/mL) (Sigma) for 20 min. The reactions were stopped by chilling on ice, adding 1 mL ice-cooled PBS/10 mmol/L EDTA and centrifugation for 5 min (4 °C, 200xg).

The cells were further processed and analyzed according to the protocol of Example 1.

Figure 4 displays a representative sample for stimulation of cells with PMA with or without IO. From this figure it emerges that challenging the cells with PMA elicited a dissimilar pattern to cells activated by FcεRI cross-linking or by fMLP. Actually, PMA clearly up-regulated expression of CD203c but elicited only poor dose-dependent expression of CD63 without clear release of histamine. In contrast, challenging the basophils of patients and control individuals with PMA-IO resulted in a significant expression of CD203c and dose-dependent CD63 expression with corresponding histamine release. Low doses of PMA-IO elicited an almost isolated up-regulation of CD203c without significant expression of CD63 or release of histamine, whereas higher doses of PMA-IO triggered a clear CD63 expression and histamine release (see lower right quadrant). In contrast, to antigen, anti-IgE or fMLP, PMA-IO-induced histamine release was not confined to the CD63^{bright+} cells but also occurred to some extent in the CD63^{dim+} cells. Ionomycin by itself did not elicit basophil activation.

The present example confirms that PMA-induced CD63 expression is rather poor or almost absent (MacGlashan, 2010, Clin Exp Allergy, 40:1365-1377), indicating that phorbol esters not to induce significant degranulation (Dvorak et al, 1992, Am J Pathol, 141:1309-1322). Adding ionomycin to PMA induces a clear dose-dependent up-regulation of CD63.

### Example 5

The present example shows the activation patterns of basophils following stimulation with IL-3.

Aliquots of 200 µL whole blood samples were challenged at 37 °C with 200 µL activation buffer (Hank's balanced salt solution (Invitrogen, Paisley, UK) with 20 µM HEPES and 7.5 % NaHCO₃, pH 7.4, without IL-3) as a negative control, 1 or 10 ng/mL interleukin (IL-)3 (PeproTech, London, England), recombinant Bet v 1 allergen (rBet v 1: 0.01 µg/mL, kindly provided by Stallergènes, Antony Cedex, France) or a combination of 0.01 µg/mL rBetv 1 and 1 or 10 ng/mL IL-3 for 20 min. The reactions were stopped by chilling on ice, adding 1 mL ice-cooled PBS/10 mmol/L EDTA and centrifugation for 5 min (4 °C, 200xg).

The cells were further processed and analyzed according to the protocol of Example 1. Figure 5 summarizes the stimulation experiments with IL-3. It is confirmed that IL-3 predominantly up-regulates CD203c without significant expression of CD63 nor release of histamine. On the contrary, the (a subpopulation of these) CD203^{bright+}CD63⁻ cells appeared to contain quantitatively more histamine than the CD203c^{dim+}CD63⁻ cells. Furthermore, as exemplified in this particular patient, IL-3 was demonstrated to facilitate basophil stimulation by allergen, as incubation of the cells by this cytokine induced shifting from CD203c up-regulation to CD203c and CD63 up-regulation.
The present example confirms that *in vitro* challenging of the basophils with IL-3 rather prime [35-42] the basophils with an up-regulation of CD203c [10;38] but without significant expression of CD63 [10;12;37] nor histamine release [35-37;43;44]. On the contrary, priming with IL-3 appears to increase intracellular histamine content.

### Example 6

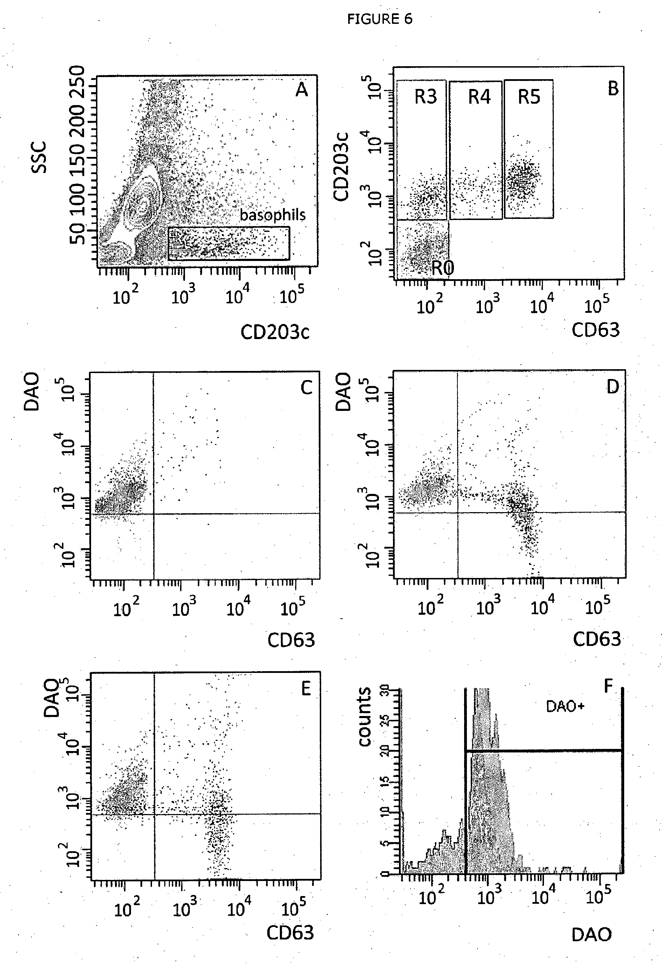

The present example describes the effect of an allergen on basophils in an allergic patient. Samples were taken after 20min activation with cephazolin sodium (Cefacidal®) in a patient suffering of profound hypotension and severe bronchospasm almost immediately after intravenous administration of cephazolin. DAO staining was performed as described herein together with CD203 and CD63 staining. The results are illustrated in Figure 6.
Basophils are characterized using side scatter (SSC)and CD203cdim+ (purple population in R2) (A). Activated basophils are defined as anti-IgE and up-regulated CD203cbright+ positive cells and co-expression of CD203cbright+ and CD63 reveals 3 subpopulations of distinct CD63 expression; i.e. CD203cbright+ CD63-(gate R3, green), CD203cbright+CD63dim+ (gate R4, blue) and CD203cbright+CD63bright+ (gate R5, red). Gate R0 denotes non-activated CD203dim+ basophils (B). Histamine-containing basophils are defined as DAO+ cells (C-F). Upon stimulation with buffer (negative control) (C), the positive control anti-IgE (D), 24% of the basophils showed histamine release (DAO- cells) and are situated in the lower-right quadrant.
Upon stimulation with cephazolin (E), 30% of the basophils demonstrated histamine release (DAO- cells (F) shows the DAO histogram (y-axis in (E)).
Thus up-regulation of activation markers CD63 and CD203c and intracellular histamine content was observed after stimulation with cephazolin.

### Example 7

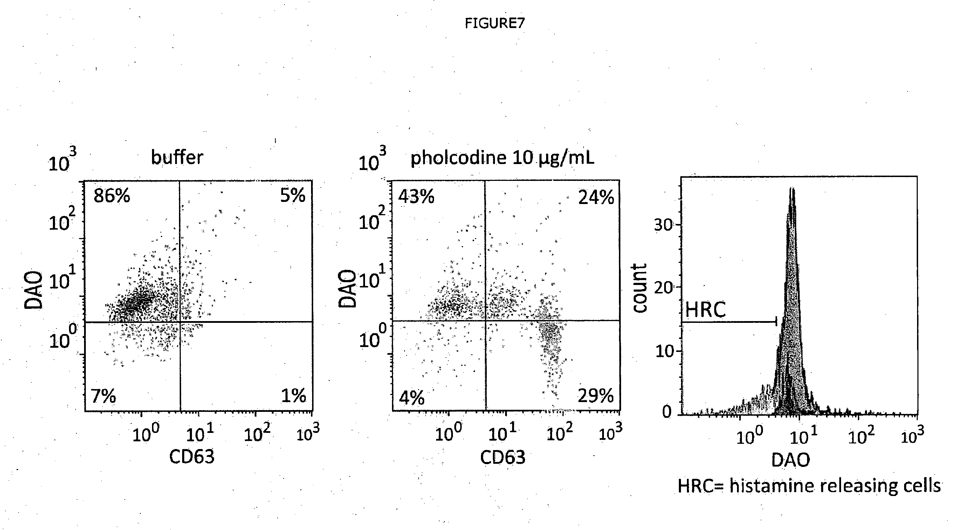

The present example further demonstrates the efficacy of the method of the invention for determining the effect of an allergen on basophils. Samples were taken after 20min activation with pholcodine in a patient suffering from generalized urticaria, angioedema and bronchospasm within 15 minutes after intake of a pholcodine-containing antitussive. DAO staining was performed as described herein together with CD203 and CD63 staining. The results are illustrated in Figure 7.

To diagnose pholcodine allergy in this patient HistaFlow was applied as follows:
Basophils are characterized using side scatter (SSC)and CD203cdim+ and plotted in CD63/DAO histogram (upper-left quadrant of left panel). Histamine-containing basophils are defined as DAO+ cells (upper quadrants of the mid panel). When stimulated with the drug pholcodine 29% of the basophils showed histamine release (DAO- cells) and are situated in the lower-right quadrant (mid panel). Twenty-four percent of cells were activated by pholcodine but did not degranulate at 20 minutes. In the histogram HRC denotes the histamine releasing cells (white), while black colored cells are activated but non-releasing and grey colored cells are the non-activated cells. Thus, up-regulation of activation markers CD63 and CD203c and intracellular histamine content was observed after contacting the sample of a patient allergic to pholcodine with pholcodine.

### Example 8

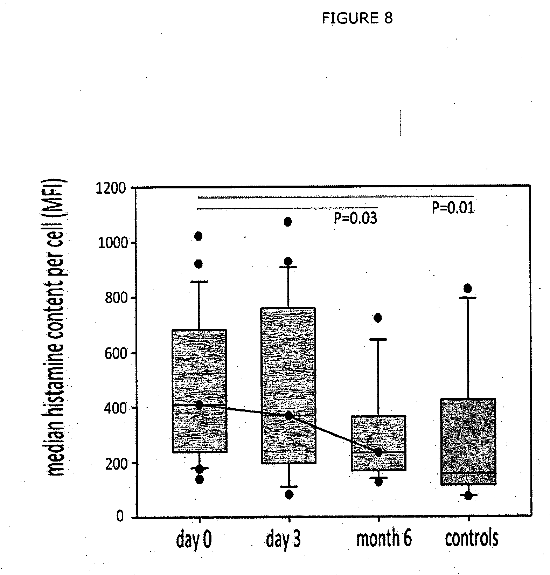

This example describes the use of the methods of the present invention for the follow-up of allergen specific immunotherapy (venom allergy)
At present there is no reliable technique to monitor the effect of allergen-specific immunotherapy in IgE-mediated venom allergy. The methods for quantifying histamine release as described herein were used to evaluate the tolerogenic effect of immunotherapy with wasp venom on the basophil responsiveness. The results are provided in Figure 8.

With respect to histamine content it was observed that before treatment (day 0) basal histamine content per cell (in resting basophils that are not activated with wasp venom) is significantly higher in patients with wasp venom allergy as compared to stung control individuals (p=0.01). This indicates a difference in histamine metabolism between patients suffering from an allergy and control individuals.

Furthermore, it is apparent from the results in Figure 8 that wasp venom therapy decreases the intracellular content in resting cells. Significance, as compared to pretreatment values (day 0) is reached after 6 months of maintenance therapy (month 6, p=0.03).

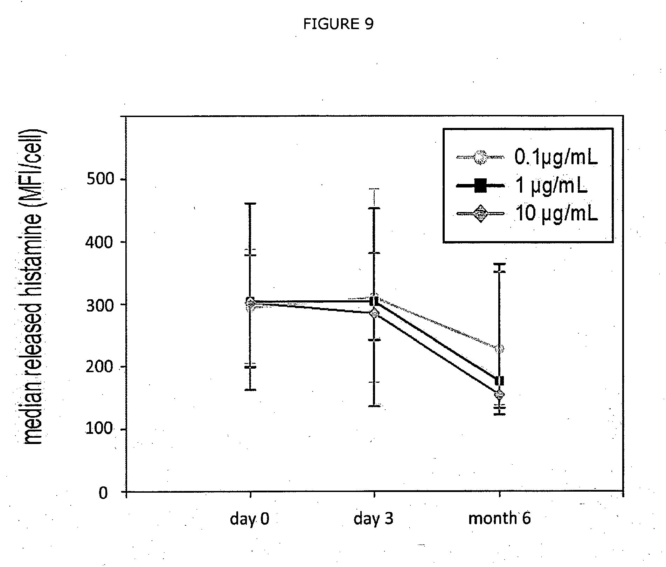

With respect to histamine release by venom-activated cells it was found that maintenance wasp venom immunotherapy (time point 3) induces a significant decrease in histamine release (Figure 9; results expressed as median released histamine (MFI/cell)).

## Claims

1. A method for quantifying histamine release or quantifying histamine in individual mast cells and basophils comprising the steps of:
a) fixing said mast cells and basophils with a fixation buffer comprising a fixation reagent,
b) permeabilizing said mast cells and basophils with permeabilization buffer comprising a permeabilization reagent,
c) staining said mast cells and basophils intracellularly with fluorochrome-labeled histaminase diamine oxidase, and
d) analyzing said mast cells and basophils in a cytometer, wherein the intracellular staining of said mast cells and basophils is indicative of the quantity of histamine present per cell in each of said mast cells and basophils.

2. The method of claim 1, which further comprises the steps of staining said cells for one or more mast cell and/or basophil selection markers; and wherein said analyzing step comprises analyzing said stained mast cells and basophils on a cytometer, wherein the signal from said fluorochrome-labeled histaminase diamine oxidase is indicative of the quantity of histamine present per cell in each of said mast cells and basophils.

3. The method of claim 2, wherein said selection marker is a cellular membrane marker selected from IgE or FcεRI.

4. The method of any one of claims 1 to 3, which further comprises the steps of staining said cells for an activation marker.

5. The method of any one of claims 2 to 4, wherein said selection and/or activation marker is identified with a fluorochrome-labeled antibody directed against said marker whereby said fluorochrome has a distinct emission spectrum from the fluorochrome-labeled histaminase diamine oxidase.

6. The method of claim 1 or 5, which further comprises the step of comparing the signal from said fluorochrome-labeled histaminase diamine oxidase to a reference value, wherein said reference value is indicative of degranulation of said mast cells and basophils.

7. The method according to any one of claims 1 to 5, wherein said fixation buffer further comprises a lysing reagent for red blood cells and/or said method further comprises, between steps (b) and (c), a step comprising the treatment of the sample with a lysing buffer.

8. The method according to any of claims 1 to 7, wherein calibration particles are added to the cells and wherein said intracellular staining of basophils and mast cells is quantified based on said calibration particles.

9. The method according to any of claims 1 to 8, further comprising the step of incubating said mast cells and basophils with an activator selected from the group comprising:
anti-IgE antibody, N-formyl-met-leu-phe (fMLP) or phorbol 12-myristate. 13-acetate/ionomycin (PMA/ionomycin), prior to mixing said incubated cells with fixation buffer.

10. A kit for quantifying intracellular histamine or quantifying histamine release in individual mast cells and basophils by cytometry comprising:
- a fluorochrome-labeled histaminase diamine oxidase;
- fixation buffer comprising a fixation reagent; and
- permeabilization buffer comprising a permeabilization reagent. And
- one or more fluorochrome-labeled antibodies directed against a selection or activation marker for mast cells and/or basophils.

11. The kit according to claim 10, further comprising one or more fluorochrome-labeled antibodies directed against CD63 or CD203c.

12. Use of the method according to any one of claims 1-9 or the kit according to claim 10 or 11 in an in vitro allergy test.

13. The use according to claim 12, wherein the allergy is an allergy to one or more compounds selected from curarising neuromuscular blocking agents, antibiotics and narcotics.

14. Use of the method according to any one of claims 1-9 or the kit according to claim 10 or 11 to determine the effect of allergen immunotherapy.

15. Use of the method according to any one of claims 1-9 or the kit according to claim 10 or 11 for testing whether a compound has an anti-allergenic effect.

## Patentansprüche

1. Verfahren zum Quantifizieren der Histaminfreisetzung oder zum Quantifizieren von Histamin in einzelnen Mastzellen und Basophilen, das die folgenden Schritte umfasst:
a) Fixieren der Mastzellen und Basophilen mit einem Fixierpuffer, der ein Fixierreagenz umfasst,
b) Permeabilisieren der Mastzellen und Basophilen mit Permeabilisierungspuffer, der ein Permeabilisierungsreagenz umfasst,
c) intrazelluläres Färben der Mastzellen und Basophilen mit fluorochrommarkierter Histaminase-Diaminoxidase, und
d) Analysieren der Mastzellen und Basophilen in einem Zytometer, wobei die intrazelluläre Färbung der Mastzellen und Basophilen die in jeder der Mastzellen und Basophilen pro Zelle vorhandene Histaminmenge anzeigt.

2. Verfahren nach Anspruch 1, das weiterhin die Schritte des Färbens der Zellen für einen oder mehrere Mastzellen- und/oder Basophilenselektionsmarker; und wobei der Schritt des Analysierens das Analysieren der gefärbten Mastzellen und Basophilen in einem Zytometer umfasst, wobei das Signal der fluorochrommarkierten Histaminase-Diaminoxidase die in jeder der Mastzellen und Basophilen pro Zelle vorhandene Histaminmenge anzeigt.

3. Verfahren nach Anspruch 2, wobei es sich bei dem Selektionsmarker um einen Zellmembranmarker, ausgewählt aus IgE oder FcεRI, handelt.

4. Verfahren nach einem der Ansprüche 1 bis 3, das weiterhin den Schritt des Färbens der Zellen für einen Aktivierungsmarker umfasst.

5. Verfahren nach einem der Ansprüche 2 bis 4, wobei der Selektions- und/oder Aktivierungsmarker mit einem fluorochrommarkierten Antikörper, der gegen diesen Marker gerichtet ist, identifiziert wird, wobei das Fluorochrom ein Emissionsspektrum aufweist, das von der fluorochrommarkierten Histaminase-Diaminoxidase unterschiedlich ist.

6. Verfahren nach Anspruch 1 oder 5, das weiterhin den Schritt des Vergleichens des Signals von der fluorochrommarkierten Histaminase-Diaminoxidase mit einem Referenzwert umfasst, wobei der Referenzwert die Degranulation der Mastzellen und Basophilen anzeigt.

7. Verfahren nach einem der Ansprüche 1 bis 5, wobei der Fixierpuffer weiterhin ein Lysereagenz für rote Blutkörperchen umfasst und/oder das Verfahren weiterhin zwischen den Schritten (b) und (c) einen Schritt umfasst, der die Behandlung der Probe mit einem Lysepuffer umfasst.

8. Verfahren nach einem der Ansprüche 1 bis 7, wobei Kalibrierungspartikel zu den Zellen hinzugefügt werden und wobei die intrazelluläre Färbung der Basophilen und Mastzellen auf Grundlage dieser Kalibrierungspartikel quantifiziert wird.

9. Verfahren nach einem der Ansprüche 1 bis 8, das weiterhin den Schritt des Inkubierens der Mastzellen und Basophilen mit einem Aktivator, ausgewählt aus der Gruppe umfassend: anti-IgE-Antikörper, N-Formyl-met-leu-phe (fMLP) oder Phorbol-12-myristat-13-acetat/ionomycin (PMA/ionomycin), vor dem Mischen der inkubierten Zellen mit Fixierpuffer, umfasst.

10. Kit zum Quantifizieren des intrazellulären Histamins oder zum Quantifizieren der Histaminfreisetzung in einzelnen Mastzellen und Basophilen mittels Zytometrie, das Folgendes umfasst:
- eine fluorochrommarkierte Histaminase-Diaminoxidase;
- Fixierpuffer, der ein Fixierreagenz umfasst; und
- Permeabilisierungspuffer, der ein Permeabilisierungsreagenz umfasst, sowie
- einen oder mehrere fluorochrommarkierte Antikörper gegen einen Selektions- oder Aktivierungsmarker für Mastzellen und/oder Basophile.

11. Kit nach Anspruch 10, das weiterhin einen oder mehrere fluorochrommarkierte Antikörper gegen CD63 oder CD203c umfasst.

12. Verwendung des Verfahrens nach einem der Ansprüche 1 bis 9 oder des Kits nach Anspruch 10 oder 11 in einem in-vitro-Allergietest.

13. Verwendung nach Anspruch 12, wobei es sich bei der Allergie um eine Allergie gegen eine oder mehrere Verbindungen, ausgewählt aus curarisierenden Muskelrelaxantien, Antibiotika und Narkotika, handelt.

14. Verwendung des Verfahrens nach einem der Ansprüche 1-9 oder des Kits nach Anspruch 10 oder 11 zur Bestimmung der Wirksamkeit der Allergenimmuntherapie.

15. Verwendung des Verfahrens nach einem der Ansprüche 1-9 oder des Kits nach Anspruch 10 oder 11 zum Testen, ob eine Verbindung eine antiallergene Wirksamkeit aufweist.

## Revendications

1. Procédé de quantification de la libération d'histamine ou de quantification d'histamine dans des mastocytes et basophiles individuels comprenant les étapes de :
a) fixation desdits mastocytes et basophiles avec un tampon de fixation comprenant un réactif de fixation,
b) perméabilisation desdits mastocytes et basophiles avec un tampon de perméabilisation comprenant un réactif de perméabilisation,
c) coloration desdits mastocytes et basophiles de façon intracellulaire avec une histaminase diamine oxydase marquée par fluorochrome, et
d) analyse desdits mastocytes et basophiles dans un cytomètre, dans lequel la coloration intracellulaire desdits mastocytes et basophiles est indicative de la quantité d'histamine présente par cellule dans chacun desdits mastocytes et basophiles.

2. Procédé de la revendication 1, qui comprend en outre les étapes de coloration desdites cellules pour un ou plusieurs marqueurs de sélection de mastocyte et/ou basophile ; et dans lequel ladite étape d'analyse comprend l'analyses desdits mastocytes et basophiles colorées sur un cytomètre, le signal de ladite histaminase diamine oxydase marquée par fluorochrome étant indicatif de la quantité d'histamine présente par cellule dans chacun desdits mastocytes et basophiles.

3. Procédé de la revendication 2, dans lequel ledit marqueur de sélection est un marqueur de membrane cellulaire choisi parmi IgE ou FcεRI.

4. Procédé de l'une quelconque des revendications 1 à 3, qui comprend en outre les étapes de coloration desdites cellules pour un marqueur d'activation.

5. Procédé de l'une quelconque des revendications 2 à 4, dans lequel ledit marqueur de sélection et/ou activation est identifié avec un anticorps marqué par fluorochrome dirigé contre ledit marqueur, ledit fluorochrome ayant un spectre d'émission distinct de l'histaminase diamine oxydase marquée par fluorochrome.

6. Procédé de la revendication 1 ou 5, qui comprend en outre l'étape de comparaison du signal de ladite histaminase diamine oxydase marquée par fluorochrome à une valeur de référence, ladite valeur de référence étant indicative de la dégranulation desdits mastocytes et basophiles.

7. Procédé selon l'une quelconque des revendications 1 à 5, dans lequel ledit tampon de fixation comprend en outre un réactif de lyse pour les érythrocytes et/ou ledit procédé comprend en outre, entre les étapes (b) et (c), une étape comprenant le traitement de l'échantillon avec un tampon de lyse.

8. Procédé selon l'une quelconque des revendications 1 à 7, dans lequel des particules d'étalonnage sont ajoutées aux cellules et dans lequel ladite coloration intracellulaire des basophiles et mastocytes est quantifiée sur la bases desdites particules d'étalonnage.

9. Procédé selon l'une quelconque des revendications 1 à 8, comprenant en outre l'étape d'incubation desdits mastocytes et basophiles avec un activateur choisi dans le groupe comprenant : un anticorps anti-IgE anticorps, N-formyl-met-leu-phe (fMLP) ou le 12-myristate,13-acétate de phorbol/ionomycine (PMA/ionomycine), avant mélange desdites cellules incubées avec le tampon de fixation.

10. Kit de quantification de l'histamine intracellulaire ou de quantification de la libération d'histamine dans des mastocytes et basophiles individuels par cytométrie comprenant :
- une histaminase diamine oxydase marquée par fluorochrome ;
- un tampon de fixation comprenant un réactif de fixation ; et
- un tampon de perméabilisation comprenant un réactif de perméabilisation ; et
- un ou plusieurs anticorps marqués par fluorochrome dirigés contre un marqueur de sélection ou d'activation pour les mastocytes et/ou basophiles.

11. Kit selon la revendication 10, comprenant en outre un ou plusieurs anticorps marqués par fluorochrome dirigés contre CD63 ou CD203c.

12. Utilisation du procédé selon l'une quelconque des revendications 1 à 9 ou kit selon la revendication 10 ou 11 dans un test d'allergie *in vitro.*

13. Utilisation selon la revendication 12, dans laquelle l'allergie est une allergie à un ou plusieurs composés choisis parmi des agents bloquants neuromusculaires curarisants, des antibiotiques et des narcotiques.

14. Utilisation du procédé selon l'une quelconque des revendications 1 à 9 ou du kit selon la revendication 10 ou 11 pour déterminer l'effet d'une immunothérapie allergénique.

15. Utilisation du procédé selon l'une quelconque des revendications 1 à 9 ou du kit selon la revendication 10 ou 11 pour déterminer si un composé a un effet anti-allergénique.
